(19) Europäisches Patentamt
European Patent Office
Office européen des brevets

(11) Veröffentlichungsnummer: **0 008 439**
**B1**

(12) **EUROPÄISCHE PATENTSCHRIFT**

(45) Veröffentlichungstag der Patentschrift: **14.04.82**

(21) Anmeldenummer: **79102981.2**

(22) Anmeldetag: **16.08.79**

(51) Int. Cl.³: **C 07 D 491/052,** C 07 D 491/147, A 61 K 31/435 // (C07D491/052, 311/00, 221/00),(C07D491/147, 311/00, 221/00),(C07D491/147, 311/00, 221/00, 209/00)

(54) **Pyrano-Heterocyclen, Verfahren zu ihrer Herstellung und diese Verbindungen enthaltende Arzneimittel.**

(30) Priorität: **21.08.78 DE 2836470**

(43) Veröffentlichungstag der Anmeldung: **05.03.80 Patentblatt 80/5**

(45) Bekanntmachung des Hinweises auf die Patenterteilung: **14.04.82 Patentblatt 82/15**

(84) Benannte Vertragsstaaten: **AT BE CH DE FR GB IT NL SE**

(56) Entgegenhaltungen: **DE-A-2 703 522**

(73) Patentinhaber: **HOECHST AKTIENGESELLSCHAFT, *Zentrale Patentabteilung Postfach 80 03 20*, D-6230 Frankfurt/Main 80 (DE)**

(72) Erfinder: **Kämmerer, Friedrich-Johannes, Dr., Am *Weiher 27, D-6203 Hochheim am Main (DE)***
Erfinder: **Gebert, Ulrich, Dr., Albert-Lortzing-Strasse 2, D-6233 Kelkheim (Taunus) (DE)**
Erfinder: **Alpermann, Hans Georg, Dr., Am Eichkopf 10, D-6240 Königstein/Taunus (DE)**

**EP 0 008 439 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Pyranoheterocyclen, Verfahren zu ihrer Herstellung und diese Verbindungen enthaltende Arzneimittel

Es ist bereits aus einer Reihe von Veröffentlichungen bekannt, dass Aniline und Tetrahydroisochinoline mit aktivierten Malonsäurederivaten im Verhältnis 1:2 zu Pyranochinolinonen bzw. Pyranobenzochinolizinonen cyclisiert werden können (vgl. E. Ziegler und H. Junek, «Mh. Chem.» 90, S. 762-767, 1959; R.E. Bowman, A. Campbell und E.M. Tanner, «J. Chem. Soc.», S. 444-447, 1959; R. Storer, D.W. Young, D.R. Taylor und J.M. Warner, «Tetrahedron» 29, S. 1721-1723, 1973; P. Venturella, A. Bellino und F. Piozzi, «Gazz. Chim. Ital.» 104, S. 297-307, 1974; E. Ziegler, H. Junek und H. Biemann, «Mh. Chem.» 92, S. 927-934, 1961). Ausserdem ist aus der deutschen Offenlegungsschrift Nr. 2703522 bekannt, dass Chinolopyran-4-on-2-carbonsäurederivate und deren Salze als Medikamente zur Behandlung von allergischem Asthma eingesetzt werden können.

Es wurde nun gefunden, dass man durch Einführung einer zusätzlichen Nitrogruppe in den Pyranoring zu Verbindungen mit wertvollen pharmakologischen Eigenschaften gelangt. Sie zeigen beispielsweise Wirkungen gegen bestimmte Immunreaktionen und zwar vor allem anaphylaktische Reaktionen (Typ I). Insbesondere besitzen sie eine ausgeprägte systemische antiallergische Wirksamkeit und eignen sich daher zur Behandlung und/oder Prophylaxe allergischer Erkrankungen.

Gegenstand der Erfindung sind somit Pyranoheterocyclen der Formel I:

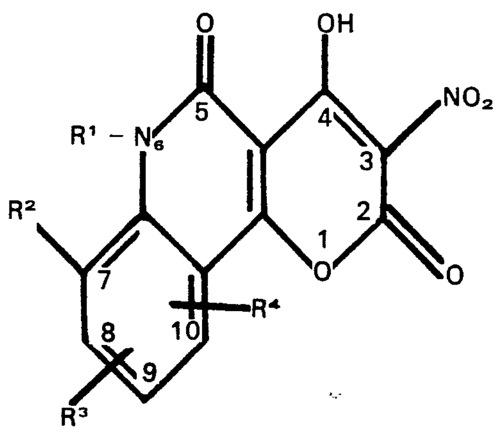

(I)

worin:

$R^1$ Wasserstoff oder Alkyl mit bis zu 4 C-Atomen,

$R^2$ Wasserstoff, Alkyl, Alkoxy oder Halogenalkyl mit jeweils bis zu 3 C-Atomen oder Halogen, oder

$R^1$ und $R^2$ zusammen eine Äthylen- oder Trimethylengruppe,

$R^3$ und $R^4$ unabhängig voneinander Wasserstoff, Alkyl oder Alkoxy mit jeweils bis zu 3 C-Atomen, die jeweils unsubstituiert, vollständig oder teilweise mit gleichen oder verschiedenen Halogenatomen (z.B. Fluor, Chlor oder Brom) substituiert sind, oder Halogen (wie Fluor, Chlor, Brom oder Jod) oder Alkoxycarbonyl mit bis zu 3 C-Atomen in der Alkylgruppe bedeuten, sowie die physiologisch verträglichen Salze dieser Verbindungen, vorzugsweise die Alkali- und Erdalkalisalze sowie die Ammoniumsalze einschliesslich solcher von organischen Basen.

Bevorzugt sind Verbindungen der Formel I und deren Salze, worin

$R^1$ und $R^2$ gemeinsam eine Trimethylengruppe bilden, und

$R^3$ und $R^4$ gleich oder verschieden sind und Wasserstoff, Alkyl oder Alkoxy mit jeweils bis zu 2 C-Atomen, Halogen (wie Fluor, Chlor oder Brom) oder die $CF_3$-Gruppe bedeuten.

Bevorzugt sind weiterhin Verbindungen der Formel I und deren Salze, worin

$R^1$ Wasserstoff oder Alkyl mit bis zu 3 C-Atomen,

$R^2$ Wasserstoff, und

$R^3$ und $R^4$ gleich oder verschieden sind und Wasserstoff, Alkyl oder Alkoxy mit jeweils bis zu 2 C-Atomen, Alkoxycarbonyl mit bis zu 3 C-Atomen in der Alkylgruppe, Halogen (wie Fluor, Chlor oder Brom) oder die $CF_3$-Gruppe bedeuten.

Die Erfindung schliesst auch die tautomeren Strukturen der Verbindungen gemäss Formel I mit ein.

Gegenstand der Erfindung ist weiterhin ein Verfahren zur Herstellung der Verbindungen der Formel I und deren Salze, worin $R^1$ bis $R^4$ die oben angegebenen Bedeutungen haben.

Es ist dadurch gekennzeichnet, dass man eine mit einem Heterocyclus kondensierte Pyranoverbindung der Formel II:

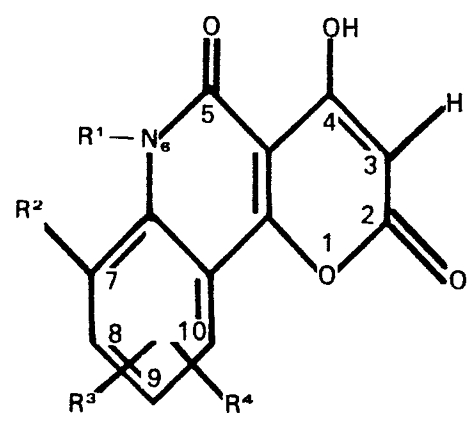

(II)

worin $R^1$, $R^2$, $R^3$ und $R^4$ die oben angegebenen Bedeutungen haben, nitriert und die erhaltenen Nitroverbindungen gegebenenfalls mit Basen in ihre physiologisch verträglichen Alkali, Erdalkali- oder Ammoniumsalze überführt.

Für die Salzbildung geeignete basische Reagentien sind beispielsweise Alkali-, bzw. Erdalkalihydroxide, -hydride, -alkoholate, -carbonate und -hydrogencarbonate sowie spezielle organische Basen, wie Äthanolamin, Diäthanolamin, Tris-(hydroxymethyl)aminomethan und N-Methylglucamin.

Die Nitrierung wird zweckmässig in Substanz oder in einem Verteilungs- oder Lösungsmittel durchgeführt, das sich unter den Reaktionsbedingungen gegenüber den Reaktionspartnern inert verhält. Hierfür kommen beispielsweise Essigsäure oder halogenierte Kohlenwasserstoffe wie Chloroform oder Tetrachlorkohlenstoff infrage. Als Nitrierungsmittel können beispielsweise die durch Einwirkung von Arsenoxid auf konzentrierte Salpetersäure entwickelten nitrosen Gase, ein Gemisch aus konzentrierter Salpetersäure und Essigsäure und rauchende oder konzentrierte Salpetersäure Verwendung finden. Bevorzugt ist die Nitrierung mit einem Gemisch aus konzentrierter Salpetersäure und Eisessig im Verhältnis 1:1 bis 1:6, vorzugsweise 1:2 bis 1:3, bei einer Temperatur zwischen 0 und 110°C, vorzugsweise zwischen 25 und 95°C. Die Reaktionszeiten betragen im allgemeinen wenige Minuten bis 2 h.

Die Ausgangsverbindungen der Formel II lassen sich herstellen, indem man ein Amin der Formel III:

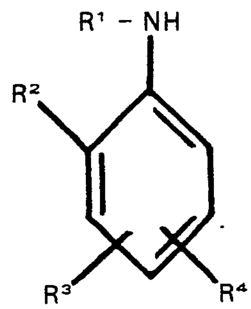

worin $R^1$, $R^2$, $R^3$ und $R^4$ die bei der allgemeinen Formel I angegebenen Bedeutungen haben, mit mindestens der doppelten molaren Menge eines Malonsäurederivats der Formel IV

$$R'OOC-CH_2-COOR' \qquad (IV)$$

umsetzt, worin R' Alkyl mit bis zu 3 C-Atomen oder Phenyl, das gegebenenfalls bis zu fünffach mit Chlor und/oder Brom substituiert ist, bedeutet.

Die Reaktion wird zweckmässig in Substanz oder in einem Verteilungs- oder Lösungsmittel durchgeführt, das sich unter den Reaktionsbedingungen gegenüber den Reaktionspartnern inert verhält. Hierfür kommen beispielsweise Kohlenwasserstoffe, wie Tetrahydronaphthalin, Dekahydronaphthalin, Naphthalin und Paraffine, aber auch Chlor- oder Brombenzol infrage. Auch ein Überschuss von Malonsäurederivaten der Formel IV kann als Reaktionsmedium dienen.

Die Verbindungen der Formel II sind tricyclisch oder, wenn $R^1$ und $R^2$ zusammen Äthylen oder Trimethylen bilden, tetracyclisch. In diesem Fall zählen die C-Atome der Äthylen- und Trimethylengruppe im Ringsystem als 7, 8 bzw. 9.

Bevorzugtes Verfahren zur Herstellung der tetracyclischen Verbindungen der Formel II, worin $R^1$ und $R^2$ gemeinsam eine Alkylenbrücke bilden, ist die Umsetzung eines Tetrahydrochinolins oder Indolins der Formel III mit dem Malonsäurediäthyl- oder bis-2,4-Dichlorphenylester, vorzugsweise ohne Verteilungs- oder Lösungsmittel, bei Temperaturen zwischen 150 und 230°C, vorzugsweise zwischen 190 und 220°C. Die Reaktionszeiten betragen im allgemeinen von wenigen Minuten bis zu 20 h. In Abhängigkeit von der Ansatzgrösse kann die Reaktion auch noch länger dauern.

Bevorzugtes Verfahren zur Herstellung der tricyclischen Verbindungen der Formel II ist die Umsetzung eines Anilins der Formel III mit dem Malonsäure-bis-2,4-dichlorphenylester in Gegenwart eines unter den Reaktionsbedingungen gegenüber den Reaktionsteilnehmern inerten Lösungs- oder Verteilungsmittels, vorzugsweise in Brombenzol oder Tetrahydronaphthalin, bei Temperaturen zwischen 150°C und dem Siedepunkt des jeweiligen Reaktionsgemisches, vorzugsweise zwischen 180 und 220°C. Die Reaktionszeiten betragen im allgemeinen von wenigen Minuten bis zu 3 h.

Im einzelnen kommen als Reste $R^1$ in Betracht Wasserstoff, Methyl, Äthyl, n- oder Isopropyl- und n-, iso- oder tert.- Butyl, wobei $R^1$ bevorzugt Alkyl mit 1 bis 3 C-Atomen darstellt. Als Reste $R^2$ kommen die gleichen Gruppen in Betracht, soweit sie bis 3 C-Atome enthalten. $R^2$ kann auch z.B. Methoxy, Äthoxy, Propoxy, Trifluormethyl, Dichlormethyl, 2,2,2-Trichloräthyl, Chlordifluormethyl sein. $R^2$ stellt jedoch bevorzugt Wasserstoff, Methyl oder Äthyl dar. $R^1$ und $R^2$ können darüberhinaus natürlich zusammen, wie bereits oben angegeben, eine Äthylen- oder Trimethylengruppe bilden.

Als Reste $R^3$ und $R^4$ kommen z.B. Wasserstoff, Methyl, Äthyl, n- oder Isopropyl, Fluor, Chlor, Brom, Jod, Trifluormethyl, Dichlormethyl, 2,2,2-Trichloräthyl, Chlordifluormethyl, Methoxy, Äthoxy, Trifluormethoxy, Propoxy, Methoxycarbonyl, Äthoxycarbonyl, Propoxycarbonyl, die Gruppe $CHClF-CF_2-O-$ in Betracht, wobei etwaige Alkyl-, Alkoxy-, Halogenalkyl-, Halogenalkoxy- und Alkoxycarbonylreste bevorzugt bis zu 2 C-Atome haben. Vorzugsweise ist einer dieser Reste von Wasserstoff verschieden. Als Verbindungen, in denen sowohl $R^3$ als auch $R^4$ eine andere Bedeutung als Wasserstoff haben, kommen z.B. die Dihalogenverbindungen, wie die Dichlor- und Dibromverbindungen, die Monohalogenmonoalkyl- oder -monoalkoxy- oder -monohalogenalkylverbindungen wie die Chlormethyl-, Chlormethoxy- und Chlortrifluormethylverbindungen in Betracht.

In Tabelle 1 sind die neuen Verbindungen der Formel II aufgeführt.

Die erfindungsgemässen Nitroverbindungen der allgemeinen Formel I und ihre physiologisch verträglichen Salze können aufgrund ihrer pharmakologischen Eigenschaften als Arzneimittel Verwendung finden, wobei man sie entweder allein, z.B. in Form von Mikrokapseln, oder vermischt mit geeigneten Trägerstoffen verabreicht. Gegenstand der Erfindung sind somit auch Arzneimittel, die aus wenigstens einer Verbindung der Formel I, gegebenenfalls in Form von deren physiologisch verträglichen Salzen, bestehen oder diesen Wirkstoff neben den üblichen, pharmazeutisch akzeptablen Träger- und/oder Verdünnungsmitteln enthalten. Die Präparate können oral, rectal oder parenteral appliziert werden, wobei die orale Anwendung bevorzugt ist. Aber auch die Inhalation fein verteilter Pulver ist eine mögliche Appliktationsform. Geeignete feste oder flüssige galenische Zubereitungsformen sind beispielsweise Granulate, Pulver, Tabletten, Kapseln, Suppositorien, Sirupe, Emulsionen, Suspensionen, Tropfen oder injizierbare Lösungen sowie Präparate mit protrahierter Wirkstofffreigabe. Als häufig verwendete Trägermittel seien z.B. Magnesiumcarbonat, verschiedene Zucker oder Stärkearten, Cellulosederivate, Gelatine, tierische und pflanzliche Öle, Polyäthylenglykole und Lösungsmittel genannt.

Eine besondere Anwendung der Verbindungen der Formel I sowie deren Salzen liegt in der Kombination mit anderen geeigneten Wirkstoffen, beispielsweise Bronchospasmolytika und Antihistaminika.

Die neuen Verfahrensprodukte der Formel I und deren Alkali- bzw. Ammoniumsalze sind in Tabelle 2 zusammengestellt. In den Beispielen beziehen sich die Verhältnisangaben jeweils auf das Volumen. Z* bedeutet Zersetzung.

*Beispiele*

1a) 11-Chlor-7,8-dihydro-2,5-dioxo-[2H,5H, 9H]-benzo-[ij]-pyrano-[2,3-b]-chinolizin-4-ol der Formel II

1 mol 6-Chlor-1,2,3,4-tetrahydrochinolin der Formel III (167,6 g) und 2 mol Malonsäure-bis-2,4-dichlorphenylester (788,1 g) werden 15 min unter Rückfluss erhitzt (ca. 220°C). Nach dem Abkühlen des Reaktionsgemisches wird das entstandene 2,4-Dichlorphenol unter vermindertem Druck im Rotationsverdampfer abdestilliert. Der zurückbleibende kristalline Brei wird in heissem Tetrahydrofuran aufgenommen und der nach dem Erkalten der Lösung entstandene kristalline Niederschlag abgesaugt, mit Isopropanol gewaschen und getrocknet. Man erhält so 185,3 g (61% der Theorie) Kristalle; Schmelzpunkt nach Umkristallisieren aus Dioxan: 283 bis 287°C (Z*).

1b) Zu derselben Verbindung gelangt man durch Umsetzung des obigen Tetrahydrochinolins mit mindestens 2 mol Malonsäurediäthylester:

0,1 mol 6-Chlor-1,2,3,4-Tetrahydrochinolin der Formel III (16,8 g) und 0,7 mol Malonsäurediäthylester (112,1 g) werden unter Rühren zum Sieden erhitzt, das dabei entstandene Äthanol abdestilliert. Die Innentemperatur der Mischung wird so langsam auf 200 bis 202°C gesteigert, dass die Reaktion in Gang bleibt und nur Äthanol überdestilliert. Nach beendeter Reaktion lässt man abkühlen, saugt den Kristallbrei ab und wäscht ihn mit Äthanol und Petroläther. Nach dem Trocknen erhält man so 27,5 g (90,5% der Theorie) Kristalle; Schmelzpunkt nach Umkristallisieren aus Dioxan: 283 bis 287°C (Z*).

Beim Einsatz von 0,22 mol Malonsäurediäthylester erhält man 22,7 g (74,9% der Theorie) kristallines Produkt; Schmelzpunkt nach Umkristallisieren aus Dioxan: 283 bis 287°C (Z*).

2. 6-Äthyl-8-chlor-5,6-dihydro-2,5-dioxo-2H-pyrano-[3,2-c]-chinolin-4-ol der Formel II

0,5 mol N-Äthyl-3-chloranilin der Formel III (77,8 g) und 1 mol Malonsäure-bis-2,4-dichlorphenylester (394,1 g) werden 15 min unter Rückfluss in 150 ml Tetrahydronaphthalin erhitzt (ca. 210°C). Man gibt 3 g Aktivkohle in die Reaktionslösung und rührt diese 10 min bei 190 bis 200°C. Anschliessend wird filtriert und das Filtrat unter vermindertem Druck im Rotationsverdampfer bis zur Trockne eingeengt. Der ölige, von Kristallen durchsetzte Rückstand (90,5 g) wird mit 2 l n-Butanol ausgekocht. Dabei geht ein Teil des Rückstandes in Lösung. Es wird heiss filtriert. Der Filterrückstand wird erst mit Isopropanol und dann mit Petroläther gewaschen und getrocknet. Man erhält so 59,2 g (40,6% der Theorie) eines kristallinen Pulvers vom Schmelzpunkt (nach Umkristallisieren aus n-Butanol) 276 bis 278°C. Die n-Butanolphase lässt man über Nacht bei 5°C stehen. Der dabei entstandene kristalline Niederschlag wird abgesaugt, gewaschen und getrocknet. Man erhält so weitere 23,6 g (16,2% der Theorie) des kristallinen Pulvers vom Schmelzpunkt 276 bis 278°C (nach Umkristallisation aus n-Butanol). Insgesamt werden so 82,8 g (56,8% der Theorie) an kristallinem Pulver vom Schmelzpunkt (nach Umkristallisation aus n-Butanol) 276 bis 278°C erhalten.

Die analog den vorstehend beschriebenen Beispielen erhaltenen Verbindungen sind in Tabelle 1 zusammengestellt.

*(Tabelle nächste Seite oben.)*

12. 11-Chlor-7,8-dihydro-3-nitro-2,5-dioxo-[2H, 5H, 9H]-benzo-[ij]-pyrano-[2,3-b]-chinolizin-4-ol der Formel I.

0,02 mol 11-Chlor-7,8-dihydro-2,5-dioxo-[2H, 5H, 9H]-benzo-[ij]-pyrano-[2,3-b]-chinolizin-4-ol aus Beispiel 1 (6,1 g) werden in 12 ml Eisessig suspendiert. Man erwärmt die Suspension auf 55°C und lässt unter Rühren 4 ml Salpetersäure (d = 1,40) so zutropfen, dass die Innentemperatur nicht über 65°C steigt.

Nach Zugabe der Salpetersäure rührt man noch 5 min bei 65°C und kühlt dann den breiigen Reaktionsansatz auf 0 bis 10°C ab. Während des Abkühlens fügt man 10 ml Wasser hinzu und saugt den Niederschlag ab, wäscht ihn zunächst mit einem Wasser/Isopropanol-Gemisch (1:1), dann mit Isopropanol und abschliessend mit Petroläther. Nach dem Trocknen erhält man 6,4 g (91,8% der Theorie) eines pulvrigen Rückstandes. Dieser Rückstand wird in 500 ml Acetonitril gelöst. Die nach dem Abkühlen ausgefallenen Kristalle werden abgesaugt, gewaschen und getrocknet. Man erhält 4,4 g (63,1% der Theorie) Kristalle vom Schmelzpunkt (nach Umkristallisieren aus Dioxan): 242 bis 244°C (Z*). Nach Einengen der Acetonitrilphase lassen sich weitere 1,4 g (20,1% der Theorie) Kristalle vom Schmelzpunkt (nach Umkristallisation aus Dioxan): 242 bis 244°C (Z*) isolieren.

13. 9-Chlor-5,6-dihydro-6-methyl-3-nitro-

Tabelle 1. Verbindungen der Formel II

| Beispiel | $R^1$ | $R^2$ | $R^3$ | $R^4$ | Schmelzpunkt (°C) |
|---|---|---|---|---|---|
| 1 | $-CH_2-CH_2-CH_2-$ | | H | 11-Cl | 283-287 (Z*) |
| 2 | $-C_2H_5$ | H | 8-Cl | H | 276-278 |
| 3 | $-CH_2-CH_2-CH_2-$ | | H | $11-CH_3$ | 274-277 (Z*) |
| 4 | $-CH_2-CH_2-CH_2-$ | | H | $11-O-CH_3$ | 296-301 |
| 5 | $-CH_2-CH_2-CH_2-$ | | 11-Cl | 12-Cl | >300 (Z*) |
| 6 | $-C_2H_5$ | H | H | 9-Cl | 239-245 |
| 7 | $-CH_3$ | H | H | 9-Cl | 282-286 |
| 8 | $-C_2H_5$ | H | H | 9-F | 214-216 |
| 9 | $-C_2H_5$ | H | H | $9-CF_3$ | 208-212 |
| 10 | $-C_2H_5$ | H | H | $9-CO_2C_2H_5$ | 221-225 |
| 11 | $-C_2H_5$ | H | 8-Cl | 9-Cl | 245-248 |

*Legende zu Tabelle 1:*

1 11-Chlor-7,8-dihydro-2,5-dioxo-[2H, 5H, 9H]-benzo-[ij]-pyrano-[2,3-b]-chinolizin-4-ol.
2 6-Äthyl-8-chlor-5,6-dihydro-2,5-dioxo-2H-pyrano-[3,2-c]-chinolin-4-ol.
3 7,8-Dihydro-11-methyl-2,5-dioxo-[2H, 5H, 9H]-benzo-[ij]-pyrano-[2,3-b]-chinolizin-4-ol.
4 7,8-Dihydro-11-methoxy-2,5-dioxo-[2H, 5H, 9H]-benzo-[ij]-pyrano-[2,3-b]-chinolizin-4-ol.
5 11,12-Dichlor-7,8-dihydro-2,5-dioxo-[2H, 5H, 9H]-benzo-[ij]-pyrano-[2,3-b]-chinolizin-4-ol.
6 6-Äthyl-9-chlor-5,6-dihydro-2,5-dioxo-2H-pyrano-[3,2-c]-chinolin-4-ol.
7 9-Chlor-5,6-dihydro-6-methyl-2,5-dioxo-2H-pyrano-[3,2-c]-chinolin-4-ol.
8 6-Äthyl-9-fluor-5,6-dihydro-2,5-dioxo-2H-pyrano-[3,2-c]-chinolin-4-ol.
9 6-Äthyl-5,6-dihydro-2,5-dioxo-9-trifluormethyl-2H-pyrano-[3,2-c]-chinolin-4-ol.
10 9-Äthoxycarbonyl-6-äthyl-5,6-dihydro-2,5-dioxo-2H-pyrano-[3,2-c]-chinolin-4-ol.
11 6-Äthyl-8,9-dichlor-5,6-dihydro-2,5-dioxo-2H-pyrano-[3,2-c]-chinolin-4-ol.

2,5-dioxo-2H-pyrano-[3,2-c]-chinolin-4-ol der Formel I.

0,02 mol 9-Chlor-5,6-dihydro-6-methyl-2,5-dioxo-2H-pyrano-[3,2-c]-chinolin-4-ol aus Beispiel 7 (5,6 g) werden in 25 ml Eisessig suspendiert. Man lässt bei 25°C unter Rühren 4 ml Salpetersäure (d = 1,40) zutropfen und erwärmt dann den breiigen Reaktionsansatz 60 min auf 95°C. Danach wird auf 20°C abgekühlt. Dabei fügt man während des Abkühlens 36 ml eines Gemisches aus Wasser und Isopropanol (1:1) zu. Es wird filtriert. Der Filterrückstand wird mit Wasser neutral gewaschen und nach dem Waschen mit Isopropanol und Petroläther getrocknet. Man erhält so 5,5 g (85% der Theorie) eines kristallinen Rückstandes, dessen Kristalle nach Umkristallisieren aus einem Isobutanol/Dimethylformamid-Gemisch (1:1) einen Schmelzpunkt von 241 bis 243°C (Z*) besitzen.

Die gemäss den Beispielen 12 und 13 hergestellten Verbindungen (Beispiele 12 bis 23) sind in Tabelle 2 zusammengefasst und ebenso auch die daraus hergestellten Salze (Beispiele 24 bis 35), deren Herstellung im einzelnen für die Beispiele 24, 29, 31 und 34 beschrieben wird.

Tabelle 2. Verbindungen der Formel I (Beispiele 12 bis 23) und deren Salze (Beispiele 24 bis 35)

| Beispiel | $R^1$ | $R^2$ | $R^3$ | $R^4$ | Schmelzpunkt (°C) |
|---|---|---|---|---|---|
| 12 | $-CH_2-CH_2-CH_2-$ | | H | 11-Cl | 242-244 (Z*) |
| 13 | $-CH_3$ | H | H | 9-Cl | 241-243 (Z*) |
| 14 | $-CH_2-CH_2-CH_2-$ | | H | H | >300 (Z*) |
| 15 | $-CH_2-CH_2-CH_2-$ | | H | $11-CH_3$ | 240-243 (Z*) |
| 16 | $-CH_2-CH_2-CH_2-$ | | H | $11-O-CH_3$ | 246-248 (Z*) |
| 17 | $-CH_2-CH_2-CH_2-$ | | 11-Cl | 12-Cl | 322-325 (Z*) |
| 18 | $-C_2H_5$ | H | H | 9-Cl | 230-232 (Z*) |
| 19 | $-C_2H_5$ | H | H | 9-F | 204-206 |
| 20 | $-C_2H_5$ | H | H | $9-CF_3$ | 196-199 |
| 21 | $-C_2H_5$ | H | H | $9-CO_2C_2H_5$ | 215-217 (Z*) |
| 22 | $-C_2H_5$ | H | 8-Cl | H | 242-244 (Z*) |
| 23 | $-C_2H_5$ | H | 8-Cl | 9-Cl | 247-248 (Z*) |

| Beispiel | Salzart | Salz der Verbindung von Beispiel | Schmelzpunkt (°C) |
|---|---|---|---|
| 24 | Na-Salz | 12 | >330 (Z*) |
| 24a | Kaliumsalz | 12 | 260-265 (Z*) |
| 24b | Äthanolaminsalz | 12 | 212-215 |

Tabelle 2 *(Forts.)*

| Beispiel | Salzart | Salz der Verbindung von Beispiel | Schmelzpunkt (°C) |
|---|---|---|---|
| 24c | Diäthanolaminsalz | 12 | 220-222 (Z*) |
| 24d | Triäthanolaminsalz | 12 | 200-205 |
| 24e | Tris-(hydroxymethyl)aminomethansalz | 12 | 222-224 |
| 25 | Na-Salz | 13 | >330 |
| 26 | Na-Salz | 15 | >340 (Z*) |
| 27 | Na-Salz | 15 | >305 (Z*) |
| 28 | Na-Salz | 16 | >260 (Z*) |
| 29 | Äthanolaminsalz | 17 | 205-207 |
| 30 | Na-Salz | 18 | >330 |
| 31 | Na-Salz | 19 | 274-276 |
| 32 | Na-Salz | 20 | >320 |
| 33 | Na-Salz | 21 | 285-288 |
| 34 | Na-Salz | 22 | 238-241 |
| 35 | Äthanolaminsalz | 23 | 212-214 |

*Legende zu Tabelle 2:*

| | |
|---|---|
| 12 + 24 + 24a-e | 11-Chlor-7,8-dihydro-3-nitro-2,5-dioxo-[2H, 5H, 9H]-benzo-[ij]-pyrano-[2,3-b]-chinolizin-4-ol und dessen Natrium-, Kalium-, Äthanolamin-, Diäthanolamin-, Triäthanolamin- und Tris-(hydroxymethyl)aminomethansalz. |
| 13 + 25 | 9-Chlor-5,6-dihydro-6-methyl-3-nitro-2,5-dioxo-2H-pyrano-[3,2-c]-chinolin-4-ol und dessen Natriumsalz. |
| 14 + 26 | 7,8-Dihydro-3-nitro-2,5-dioxo-[2H, 5H, 9H]-benzo-[ij]-pyrano-[2,3-b]-chinolizin-4-ol und dessen Natriumsalz. |
| 15 + 27 | 7,8-Dihydro-11-methyl-3-nitro-2,5-dioxo-[2H, 5H, 9H]-benzo-[ij]-pyrano-[2,3-b]-chinolizin-4-ol und dessen Natriumsalz. |
| 16 + 28 | 7,8-Dihydro-11-methoxy-3-nitro-2,5-dioxo-[2H, 5H, 9H]-benzo-[ij]-pyrano-[2,3-b]-chinolizin-4-ol und dessen Natriumsalz. |
| 17 + 29 | 11,12-Dichlor-7,8-dihydro-3-nitro-2,5-dioxo-[2H, 5H, 9H]-benzo-[ij]-pyrano-[2,3-b]-chinolizin-4-ol und dessen Äthanolaminsalz. |
| 18 + 30 | 6-Äthyl-9-chlor-5,6-dihydro-3-nitro-2,5-dioxo-2H-pyrano-[3,2-c]-chinolin-4-ol und dessen Natriumsalz. |
| 19 + 31 | 6-Äthyl-9-fluor-5,6-dihydro-3-nitro-2,5-dioxo-2H-pyrano-[3,2-c]-chinolin-4-ol und dessen Natriumsalz. |
| 20 + 32 | 6-Äthyl-5,6-dihydro-3-nitro-2,5-dioxo-9-trifluormethyl-2H-pyrano-[3,2-c]-chinolin-4-ol und dessen Natriumsalz. |
| 21 + 33 | 9-Äthoxycarbonyl-6-äthyl-5,6-dihydro-3-nitro-2,5-dioxo-2H-pyrano-[3,2-c]-chinolin-4-ol und dessen Natriumsalz. |
| 22 + 34 | 6-Äthyl-8-chlor-5,6-dihydro-3-nitro-2,5-dioxo-2H-pyrano-[3,2-c]-chinolin-4-ol und dessen Natriumsalz. |
| 23 + 35 | 6-Äthyl-8,9-dichlor-5,6-dihydro-3-nitro-2,5-dioxo-2H-pyrano-[3,2-c]-chinolin-4-ol und dessen Äthanolaminsalz. |

*Herstellung von Salzen:*

24. 11-Chlor-7,8-dihydro-3-nitro-2,5-dioxo-[2H, 5H, 9H]-benzo-[ij]-pyrano-[2,3-b]-chinolizin-4-olnatriumsalz.

0,1 mol 11-Chlor-7,8-dihydro-3-nitro-2,5-dioxo-[2H, 5H, 9H]-benzo-[ij]-pyrano-[2,3-b]-chinolizin-4-ol aus Beispiel 12 (34,9 g) werden in 1100 ml Wasser suspendiert. Unter Rühren fügt man zunächst 20 ml Aceton und anschliessend soviel einer 1 N Natronlauge bei, dass der pH-Wert der Lösung 7,0 beträgt. Man erhitzt den Ansatz auf 85°C und filtriert. Die aus dem Filtrat beim Abkühlen ausgefallenen Kristalle werden abgesaugt, mit Äthanol und Petroläther gewaschen und getrocknet. Man erhält so 30,2 g (81% der Theorie) Kristalle vom Schmelzpunkt (nach Umkristallisieren aus Wasser): >330°C (Z*).

29. 11,12-Dichlor-7,8-dihydro-3-nitro-2,5-dioxo-[2H, 5H, 9H]-benzo-[ij]-pyrano-[2,3-b]-chinolizin-4-oläthanolaminsalz.

Zu einer Suspension von 0,05 mol 11,12-Dichlor-7,8-dihydro-3-nitro-2,5-dioxo-[2H, 5H, 9H]-benzo-[ij]-pyrano-[2,3-b]-chinolizin-4-ol aus Beispiel 17 (19,2 g) in 900 ml Äthanol lässt man 0,05 Mol Äthanolamin (3,05 g) in der Siedehitze zutropfen. Man lässt weitere 15 min unter Rückfluss sieden und kühlt auf Zimmertemperatur ab. Der entstandene Niederschlag wird abgesaugt, mit Äthanol gewaschen und aus Methanol umkristallisiert. Man erhält so 17,2 g (77,5% der Theorie) eines hellbraunen kristallinen Pulvers mit dem Schmelzpunkt (nach Umkristallisieren aus Dimethylacetamid/Methanol): 205 bis 207°C (Z*).

31. 6-Äthyl-9-fluor-5,6-dihydro-3-nitro-2,5-dioxo-2H-pyrano-[3,2-c]-chinolin-4-olnatriumsalz.

0,05 mol 6-Äthyl-9-fluor-5,6-dihydro-3-nitro-2,5-dioxo-2H-pyrano-[3,2-c]-chinolin-4-ol aus Beispiel 19 (16 g) werden in 250 ml Wasser suspendiert. Man fügt 15 ml Aceton hinzu und anschliessend soviel einer 1 N Natriumhydrogencarbonatlösung, dass der pH-Wert der Lösung 7,0 bis 7,5 beträgt. Gegen Ende der Salzbildung erwärmt man auf 70 bis 80°C, filtriert und saugt die beim Abkühlen ausgefallenen Kristalle ab. Man wäscht mit Wasser und trocknet sie. Man erhält so 14,6 g (85% der Theorie) Kristalle vom Schmelzpunkt (nach Umkristallisieren aus Wasser): 274 bis 276°C.

34. 6-Äthyl-8-chlor-5,6-dihydro-3-nitro-2,5-dioxo-2H-pyrano-[3,2-c]-chinolin-4-ol-natriumsalz.

0,05 mol 6-Äthyl-8-chlor-5,6-dihydro-3-nitro-2,5-dioxo-2H-pyrano-[3,2-c]-chinolin-4-ol aus Beispiel 22 (16,8 g) werden in 75 ml Dimethylacetamid gelöst. Diese Lösung lässt man unter Rühren in eine Suspension von 1,5 g einer 80%igen Natriumhydridweissölsuspension (Merck) in 25 ml Dimethylacetamid tropfen. Nach beendeter Wasserstoffentwicklung lässt man weitere 15 min bei 40 bis 50°C rühren. Man lässt abkühlen, filtriert bei 25°C und versetzt das Filtrat unter Rühren mit 750 ml eines Methyläthylketon/Methanol-Gemisches (2:1). Es wird kurz unter Rückfluss erhitzt und abgekühlt. Dabei fällt das Natriumsalz in kristalliner und analysenreiner Form aus. Die Kristalle werden abgesaugt, gewaschen und getrocknet. Man erhält so 15,4 g (86% der Theorie) Kristalle vom Schmelzpunkt: 238 bis 241°C.

*Pharmakologische Prüfung und Ergebnisse*

Die erfindungsgemässen Verbindungen der Formel I sowie deren Salze zeigen bei guter Verträglichkeit starke antiallergische Eigenschaften sowohl nach intravenöser als auch nach oraler Gabe in den Modellen der passiven cutanen Anaphylaxie (PCA) an Ratten.

Nach der Methode von J. Goose und A.M.J.N. Blair, „Immunology", *16*, 749, 1969 wird an Sprague-Dawley-Ratten eine PCA durch intracutane Applikation von homologem Antiserum, 0,1 ml einer Verdünnung von 1:16 bzw. 1:32 in beide Flanken, vorbereitet. Das Antiserum ist gegen Ovalbumin gerichtet und wird in Ratten durch kombinierte Gabe von Ovalbumin mit *Bordetella pertussis* als Adjuvans und Boosterung mit *Nippostrongylus*-Larven hergestellt. 72 h nach der intracutanen Antiserumgabe erfolgt zur Auslösung der cutanen anaphylaktischen Reaktion die intravenöse Injektion des Antigens, das aus einem Gemisch von je 25 mg/kg Ovalbumin und Evans-Blue besteht. Die Verbindung der Formel I bzw. deren Salze werden bei intravenöser Applikation unmittelbar vor Ovalbumin/Farbstoff-Gemisch, bei oraler Prüfung 30 min vorher, gegeben. 10 min nach der Ovalbumin/Farbstoff-Injektion werden die Tiere getötet und der Durchmesser der Farbstoffausbreitung wird in der Unterhaut gemessen.

Als Vergleichssubstanz dient das Standardpräparat Dinatriumchromoglycat (DSCG).

In den nachstehenden Tabellen 3 und 4 sind die Ergebnisse des passiven cutanen Anaphylaxie-Tests (PCA) zusammengestellt.

Tabelle 3. Hemmung der PCA an Ratten bei i.v.-Behandlung

| Verbindung aus Beispiel | n* | Dosis (mg/kg) | Hemmung gegen Kontrolle (Verdünnung 1:16) (%) |
|---|---|---|---|
| 24 (12) | 6 | 0,1 | 12 |
| | | 0,3 | 50 |
| | | 1,0 | 89 |
| | | 3,0 | 100 |
| 25 (13) | 5 | 0,01 | 44 |
| | | 0,03 | 67 |
| | | 0,1 | 100 |
| 30 (18) | 5 | 0,03 | 50 |
| | | 0,1 | 88 |
| | | 0,3 | 100 |
| 31 (19) | 5 | 0,01 | 27 |
| | | 0,03 | 60 |
| | | 0,1 | 80 |
| | | 0,3 | 87 |
| | | 3,0 | 100 |
| 32 (20) | 5 | 0,03 | 19 |
| | | 0,1 | 69 |
| 32 (20) | 5 | 0,3 | 75 |
| | | 3,0 | 100 |
| 35 (23) | 5 | 0,1 | 20 |
| | | 0,3 | 53 |
| | | 1,0 | 73 |
| | | 30,0 | 100 |
| DSCG als Vergleich | 6 | 0,1 | 9 |
| | | 0,3 | 34 |
| | | 1,0 | 71 |

* n bedeutet die Anzahl der Tiere/Dosis.

Die in Klammern gesetzten Zahlen der ersten Spalte bezeichnen die Beispielnummern der jeweils zugehörigen Stammverbindung.

Tabelle 4. Hemmung der PCA an Ratten bei oraler Behandlung

| Verbindung aus Beispiel | n* | Dosis (mg/kg) | Hemmung gegen Kontrolle bei Verdünnung des Antiserums (%) 1:16 | 1:32 |
|---|---|---|---|---|
| 24 | 6 | 0,3 | 22 | 27 |
| | | 1,0 | 50 | 73 |
| | | 3,0 | 44 | 73 |
| | | 10,0 | 44 | 73 |
| DSCG als Vergleich | 6 | 10,0 | 0 | 0 |
| | | 30,0 | 5 | 0 |

* n bedeutet die Anzahl der Tiere/Dosis.

Wie aus den Tabellen 3 und 4 ersichtlich ist, zeigen die erfindungsgemässen Verbindungen nach parenteraler Verabreichung eine wesentlich stärkere antianaphylaktische Wirkung als das Vergleichspräparat DSCG. Ein für die Therapie besonders wichtiger Vorteil der erfindungsgemässen Verbindungen gegenüber dem oral inaktiven DSCG besteht darin, dass sie auch nach oraler Verabreichung hoch wirksam sind.

**Patentansprüche für die Vertragsstaaten:**

BE, CH, DE, FR, GB, IT, NL, SE

1. Pyranoheterocyclen der Formel I:

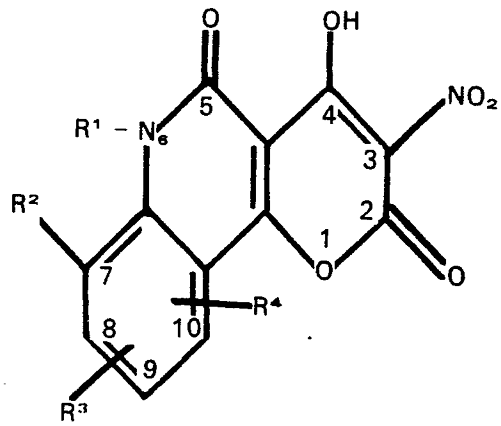

(I)

worin

$R^1$ Wasserstoff oder Alkyl mit bis zu 4 C-Atomen,

$R^2$ Wasserstoff, Alkyl, Alkoxy oder Halogenalkyl mit jeweils bis zu 3 C-Atomen oder Halogen, oder

$R^1$ und $R^2$ zusammen eine Äthylen- oder Trimethylengruppe, und

$R^3$ und $R^4$ unabhängig voneinander Wasserstoff, Alkyl oder Alkoxy mit jeweils bis zu 3 C-Atomen, die jeweils unsubstituiert, vollständig oder teilweise mit gleichen oder verschiedenen Halogenatomen substituiert sind, Halogen oder Alkoxycarbonyl mit bis zu 3 C-Atomen in der Alkylgruppe bedeuten, sowie die physiologisch verträglichen Salze dieser Verbindungen.

2. Verbindungen der Formel I von Anspruch 1 und deren Salze, dadurch gekennzeichnet, dass

$R^1$ und $R^2$ gemeinsam eine Trimethylengruppe bilden, und

$R^3$ und $R^4$ gleich oder verschieden sind und Wasserstoff, Alkyl oder Alkoxy mit jeweils bis zu 2 C-Atomen, Halogen oder die $CF_3$-Gruppe bedeuten.

3. Verbindungen der Formel I von Anspruch 1 und deren Salze, dadurch gekennzeichnet, dass

$R^1$ Wasserstoff oder Alkyl mit bis zu 3 C-Atomen,

$R^2$ Wasserstoff, und

$R^3$ und $R^4$ gleich oder verschieden sind und Wasserstoff, Alkyl oder Alkoxy mit jeweils bis zu 2 C-Atomen, Alkoxycarbonyl mit bis zu 3 C-Atomen in der Alkylgruppe, Halogen oder die $CF_3$-Gruppe bedeuten.

4. Verfahren zur Herstellung von Verbindungen der Formel I von Anspruch 1 und deren Salzen dadurch gekennzeichnet, dass man eine Verbindung der Formel:

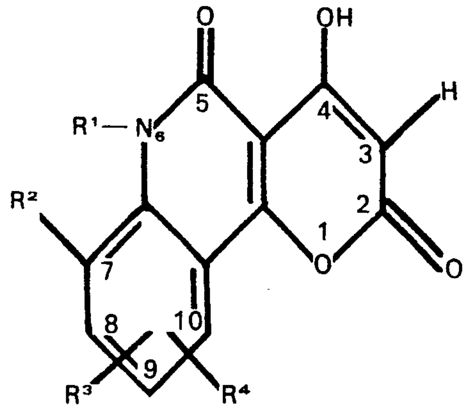

(II)

worin

$R^1$ bis $R^4$ die im Anspruch 1 angegebenen Bedeutungen haben, in an sich bekannter Weise nitriert und die erhaltene Nitroverbindung der Formel I gegebenenfalls mit Basen in physiologisch verträgliche Salze überführt.

5. Verfahren nach Anspruch 4, dadurch gekennzeichnet, dass man die Nitrierung der Verbindungen gemäss Formel II in einem Gemisch aus konzentrierter Salpetersäure und Eisessig im Volumenverhältnis 1:1 bis 1:6 bei Temperaturen zwischen 0 und 110°C durchführt.

6. Verfahren nach einem der beiden Ansprüche 4 oder 5 zur Herstellung von Verbindungen der Formel I, dadurch gekennzeichnet, dass man den Ausgangsstoff der Formel (II) durch Umsetzung eines Amins der Formel:

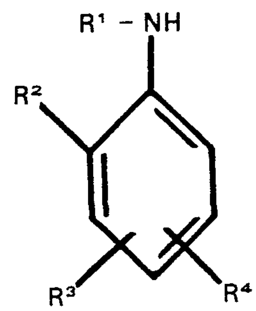

(III)

in an sich bekannter Weise mit mindestens der doppelten molaren Menge eines Malonsäurederivats der Formel $R'OOC-CH_2-COOR'$ (IV) herstellt, wobei in den Formeln III und IV $R^1$, $R^2$, $R^3$ und $R^4$ die oben genannten Bedeutungen haben und R' für Alkyl mit bis zu 3 C-Atomen oder Phenyl steht, das bis zu fünffach mit Chlor und/oder Brom substituiert sein kann.

7. Verfahren nach Anspruch 6, dadurch gekennzeichnet, dass man zur Herstellung der tetracyclischen Ausgangsverbindungen gemäss Formel II, worin $R^1$ und $R^2$ gemeinsam eine Alkylenbrücke bilden, $R^3$ und $R^4$ die im Anspruch 4 angegebenen Bedeutungen haben, bevorzugt ohne Verteilungs- oder Lösungsmittel, bei Temperaturen zwischen 150 und 230°C, vorzugsweise zwischen 190 und 220°C, arbeitet.

8. Verfahren nach Anspruch 6, dadurch gekennzeichnet, dass man bei der Herstellung der tricyclischen Ausgangsverbindungen gemäss Formel II, bevorzugt in Gegenwart eines unter den Reaktionsbedingungen gegenüber den Reaktionsteilnehmern inerten Lösungs- oder Verteilungsmittels bei Temperaturen zwischen 150°C und dem Siedepunkt des jeweiligen Reaktionsgemisches, vorzugsweise bei Temperaturen zwischen 180 und 220°C, arbeitet.

9. Verfahren nach einem oder mehreren der Ansprüche 4 bis 8 zur Herstellung von Salzen der Verbindungen gemäss Formel I, dadurch gekennzeichnet, dass man die physiologisch verträglichen Alkali-, Erdalkali- oder Ammoniumsalze bildet.

10. Arzneimittel, gekennzeichnet durch einen Gehalt an — oder bestehend aus — mindestens einer Verbindung der Formel I nach Ansprüchen 1 bis 3 und/oder deren Salzen oder mindestens einer nach dem Verfahren gemäss den Ansprüchen 4 bis 9 hergestellten Verbindungen der Formel I.

**Revendications pour les Etats contractants:**

BE, CH, DE, FR, GB, IT, NL, SE

1. Composés pyrannohétérocycliques de formule I:

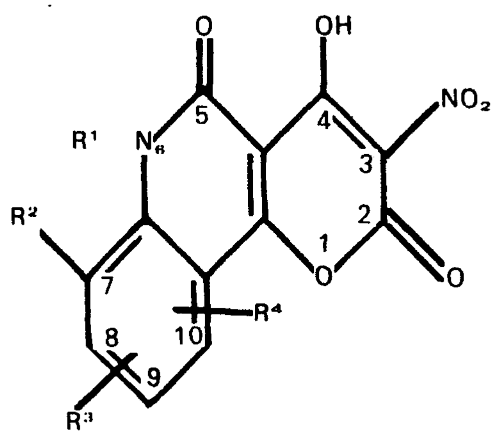

(I)

dans laquelle:

$R^1$ représente l'hydrogène ou un alkyle ayant jusqu'à 4 atomes de carbone,

$R^2$ représente l'hydrogène ou un alkyle, un alcoxy ou un halogénoalkyle ayant chacun jusqu'à 3 atomes de carbone ou un halogène, ou

$R^1$ et $R^2$ forment ensemble un groupe éthylène ou triméthylène, et

$R^3$ et $R^4$ représentent indépendamment l'un de l'autre l'hydrogène, un alkyle ou un alcoxy ayant chacun jusqu'à 3 atomes de carbone, qui sont non-substitués, totalement ou partiellement substitués avec des atomes d'halogène identiques ou différents, un halogène ou un alcoxycarbonyle ayant jusqu'à 3 atomes de carbone dans le groupe alkyle, ainsi que les sels physiologiquement acceptables de ces composés.

2. Composés de formule I selon la revendication 1 et leurs sels, caractérisés en ce que:

$R^1$ et $R^2$ forment ensemble un groupe triméthylène, et

$R^3$ et $R^4$ sont identiques ou différents et représentent l'hydrogène, un alkyle ou un alcoxy ayant chacun jusqu'à 2 atomes de carbone, un halogène ou le groupe $CF_3$.

3. Composés de formule I selon la revendication 1 et leurs sels, caractérisés en ce que:

$R^1$ représente l'hydrogène ou un alkyle ayant jusqu'à 3 atomes de carbone,

$R^2$ représente l'hydrogène, et

$R^3$ et $R^4$ sont identiques ou différents et représentent l'hydrogène, un alkyle ou un alcoxy ayant chacun jusqu'à 2 atomes de carbone, un alcoxycarbonyle ayant jusqu'à 3 atomes de carbone dans le groupe alkyle, un halogène ou le groupe $CF_3$.

4. Procédé de préparation des composés de formule I selon la revendication 1 et leurs sels, caractérisé en ce qu'on nitre d'une manière connue en soi un composé de formule:

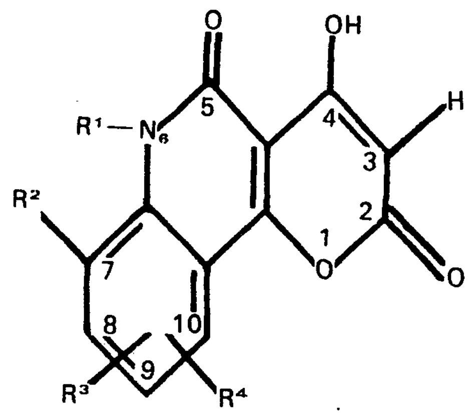

(II)

dans laquelle $R^1$ à $R^4$ ont les significations indiquées dans la revendication 1, et on transforme éventuellement le composé nitro de formule I obtenu, avec des bases, en des sels physiologiquement acceptables.

5. Procédé selon la revendication 4, caractérisé en ce qu'on effectue la nitration des composés de formule II dans un mélange d'acide nitrique concentré et d'acide acétique dans un rapport volumique de 1:1 à 1:6 à des températures comprises entre 0 et 110°C.

6. Procédé selon l'une des revendications 4 ou 5 pour la préparation de composés de formule I, caractérisé en ce qu'on prépare la substance de départ de formule II par réaction d'une amine de formule:

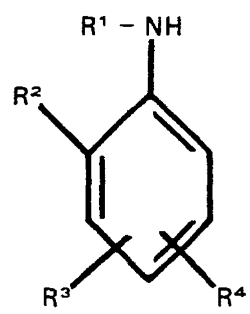

(III)

d'une manière connue en soi, avec au moins une quantité deux fois molaire d'un dérivé de l'acide malonique de formule:

$$R'OOC-CH_2-COOR' \qquad (IV)$$

auquel cas dans les formules III et IV, $R^1$, $R^2$, $R^3$ et $R^4$ ont les significations indiquées ci-dessus et R' représente un alkyle ayant jusqu'à 3 atomes de carbone ou un phényle qui peut être substitué jusqu'à 5 fois avec du chlore et/ou du brome.

7. Procédé selon la revendication 6, caractérisé en ce que, pour préparer les composés de départ tétracycliques de formule II, où $R^1$ et $R^2$ forment ensemble un pont alkylène et $R^3$ et $R^4$ ont les significations indiquées dans la revendication 4, on travaille de préférence sans agent de répartition ou solvant, à des températures comprises entre 150 et 230°C, de préférence entre 190 et 220°C.

8. Procédé selon la revendication 6, caractérisé en ce que pour préparer les composés de départ tricycliques de formule II, on travaille de préférence en présence d'un solvant ou agent de répartition inerte vis-à-vis des partenaires de réaction dans les conditions de la réaction, à des températures comprises entre 150°C et le point d'ébullition du mélange correspondant, de préférence à des températures comprises entre 180 et 220°C.

9. Procédé selon une ou plusieurs des revendications 4 à 8, pour la préparation des sels des composés de formule I, caractérisé en ce qu'on forme les sels alcalins, alcalino-terreux ou d'ammonium physiologiquement compatibles.

10. Médicament, caractérisé par une teneur en, ou constitués de, au moins un composé de formule I selon l'une quelconque des revendications 1 à 3 et/ou leurs sels ou au moins un des composés de formule I préparés par le procédé selon l'une quelconque des revendications 4 à 9.

**Claims for the contracting States:**

BE, CH, DE, FR, GB, IT, NL, SE

1. Pyranoheterocycles of the formula I:

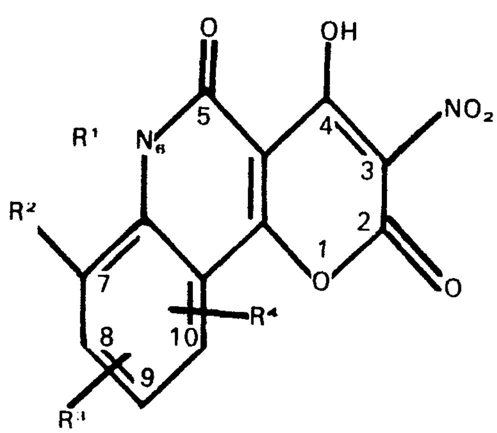

(I)

wherein
$R^1$ is hydrogen or alkyl having 1 to 4 carbon atoms,
$R^2$ is hydrogen, alkyl, alkoxy or haloalkyl each having up to 3 carbon atoms, or halogen, or
$R^1$ and $R^2$ together form an ethylene or trimethylene group, and
$R^3$ and $R^4$, are, independently from each other, hydrogen, alkyl or alkoxy each having up to 3 carbon atoms being unsubstituted or completely or partially substituted, with the same or different halogen atoms; halogen or alkoxycarbonyl having up to 3 carbon atoms in the alkyl group and the physiologically acceptable salts thereof.

2. Compounds of formula I of claim 1 and their salts, characterised in that
$R^1$ and $R^2$ together form a trimethylene group, and
$R^3$ and $R^4$ are identical or different and mean hydrogen, alkyl or alkoxy each with up to 2 carbon atoms, halogen or the $CF_3$-group.

3. Compounds of formula I of claim 1 and their salts, characterised in that
$R^1$ is hydrogen or alkyl with up to 3 carbon atoms,
$R^2$ is hydrogen, and
$R^3$ and $R^4$ are identical or different and mean hydrogen, alkyl or alkoxy each with up to 2 carbon atoms, alkoxycarbonyl with up to 3 carbon atoms in the alkyl moiety, halogen or the $CF_3$-group.

4. Process for the preparation of compounds of formula I of claim 1 and of their salts, characterised in that a compound of formula II:

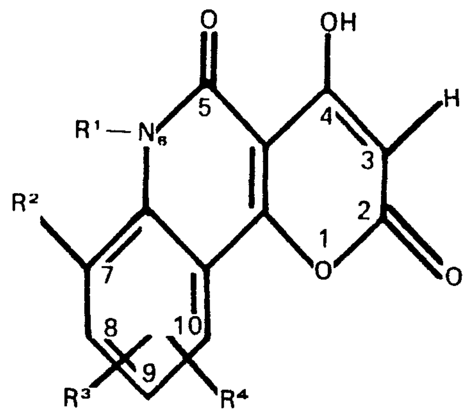

(II)

wherein $R^1$ to $R^4$ have the meanings given in claim 1, is nitrated in a principally known manner and the obtained nitro compound of formula I is optionally converted with bases into their physiologically acceptable salts.

5. Process as claimed in claim 4, characterised in that the nitration of the compounds of formula II is performed in a mixture of concentrated nitric acid and glacial acetic acid in a volume ratio of 1:1 to 1:6 at temperatures between 0 and 110°C.

6. Process as claimed in claim 4 or 5 for the preparation of compounds of formula I, characterised in that the starting substance of the formula II is prepared by reacting an amine of the formula III:

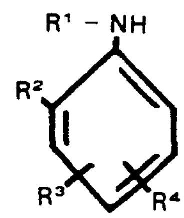

(III)

in a principally known manner, with at least the double molar quantity of a malonic acid derivative of formula:

$$R'OOC-CH_2-COOR' \qquad (IV)$$

$R^1$, $R^2$, $R^3$ and $R^4$ in the formulae III and IV having the meanings given above and R' stands for alkyl with up to 3 carbon atoms or phenyl, which may be substituted up to 5 times with chlorine and/or bromine.

7. Process as claimed in claim 6, characterised in that the tetracyclic starting compounds of the formula II, in which $R^1$ and $R^2$ together form an alkylene bridge and $R^3$ and $R^4$ have the meanings given in claim 4, are prepared, preferably without dispersing agents or solvents, at temperatures between 150 and 230°C, preferably between 190 and 220°C.

8. Process as claimed in claim 6, characterised in that the tricyclic starting compounds of the formula II are prepared, preferably in the presence of a solvent or dispersing agent which is inert toward the reactants, at temperatures between 150°C and the boiling point of the respective reaction mixture, preferably at temperatures between 180 and 220°C.

9. Process as claimed in one or several claims 4 to 8, for the preparation of salts of the compounds of formula I, characterised in that the physiologically acceptable alkali metal, alkaline earth metal or ammonium salts are formed.

10. Medicament, characterised by a content of, or consisting of, at least one compound of the formula I as claimed in claims 1 to 3 and/or their salts or at least one of the compounds of the formula I prepared according to claims 4 to 9.

**Patentansprüche für den Vertragsstaat:**

AT

1. Verfahren zur Herstellung von Nitroverbindungen von Pyranoheterocyclen der Formel:

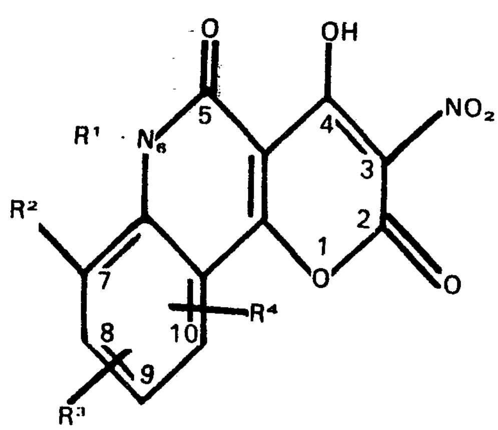

(I)

sowie physiologisch verträglicher Salze dieser Verbindungen, dadurch gekennzeichnet, dass man Verbindungen der Formel:

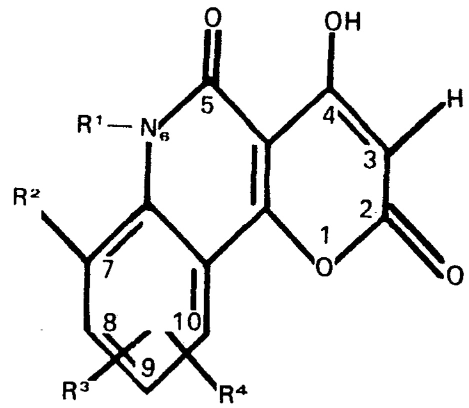

(II)

nitriert und die erhaltenen Nitroverbindungen gegebenenfalls mit Basen in physiologisch verträgliche Salze überführt,
wobei in obigen Formeln
$R^1$ Wasserstoff oder Alkyl mit bis zu 4 C-Atomen,
$R^2$ Wasserstoff, Alkyl, Alkoxy oder Halogenalkyl mit jeweils bis zu 3 C-Atomen oder Halogen, oder
$R^1$ und $R^2$ zusammen eine Äthylen- oder Trimethylengruppe, und
$R^3$ und $R^4$ unabhängig voneinander Wasserstoff, Alkyl oder Alkoxy mit jeweils bis zu 3 C-Atomen, die jeweils unsubstituiert, vollständig oder teilweise mit gleichen oder verschiedenen Halogenatomen substituiert sind, Halogen, Alkoxycarbonyl mit bis zu 3 C-Atomen in der Alkylgruppe bedeuten.

2. Verfahren nach Anspruch 1, dadurch gekennzeichnet, dass man die Nitrierung der Verbindungen gemäss Formel II in einem Gemisch aus konzentrierter Salpetersäure und Eisessig im Volumenverhältnis 1:1 bis 1:6 bei Temperaturen zwischen 0 und 110°C durchführt.

3. Verfahren nach einem der beiden Ansprüche 1 oder 2, dadurch gekennzeichnet, dass solche Verbindungen der Formel I hergestellt werden, worin
$R^1$ und $R^2$ gemeinsam eine Trimethylengruppe bilden, und
$R^3$ und $R^4$ gleich oder verschieden sind und Wasserstoff, Alkyl oder Alkoxy mit jeweils bis zu 2 C-Atomen, Halogen oder die $CF_3$-Gruppe bedeuten.

4. Verfahren nach einem der beiden Ansprüche 1 oder 2, dadurch gekennzeichnet, dass solche Verbindungen der Formel I hergestellt werden, worin
$R^1$ Wasserstoff oder Alkyl mit bis zu 3 C-Atomen,
$R^2$ Wasserstoff, und
$R^3$ und $R^4$ gleich oder verschieden sind und Wasserstoff, Alkyl oder Alkoxy mit jeweils bis zu 2 C-Atomen, Alkoxycarbonyl mit bis zu 3 C-Atomen in der Alkylgruppe, Halogen oder die $CF_3$-Gruppe bedeuten.

5. Verfahren nach einem oder mehreren der Ansprüche 1 bis 4, dadurch gekennzeichnet, dass 11-Chlor-7,8-dihydro-3-nitro-2,5-dioxo-[2H, 5H,9H]-benzo-[ij]-pyrano-[2,3-b]-chinolizin-4-olnatriumsalz hergestellt wird.

6. Verfahren nach einem oder mehreren der Ansprüche 1 bis 5 zur Herstellung von Salzen der Verbindungen gemäss Formel I, dadurch gekennzeichnet, dass man die physiologisch verträglichen Alkali-, Erdalkali- oder Ammoniumsalze bildet.

7. Verfahren nach einem oder mehreren der Ansprüche 1 bis 5, dadurch gekennzeichnet, dass man die Ausgangsverbindungen der Formell II durch Umsetzung eines Amins der allgemeinen Formel:

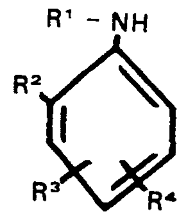

(III)

in an sich bekannter Weise mit mindestens der doppelten molaren Menge eines Malonsäurederivats der Formel $R'OOC-CH_2COOR'$ (IV) herstellt, worin in den Formeln II und III $R^1$, $R^2$, $R^3$ und $R^4$ die oben genannten Bedeutungen haben, und R' für Alkyl mit bis zu 3 C-Atomen oder Phenyl steht, das bis zu fünffach mit Chlor und/oder Brom substituiert sein kann.

8. Verfahren nach Anspruch 7, dadurch gekennzeichnet, dass die tetracyclischen Ausgangsverbindungen gemäss Formel II, worin $R^1$ und $R^2$ gemeinsam eine Alkylenbrücke bilden, bevorzugt ohne Verteilungs- oder Lösungsmittel, durch Umsetzung bei Temperaturen zwischen 150 und 230°C, vorzugsweise zwischen 190 und 220°C, hergestellt werden.

9. Verfahren nach Anspruch 7, dadurch gekennzeichnet, dass die tricyclischen Ausgangsverbindungen gemäss Formel II, bevorzugt in Gegenwart eines unter den Reaktionsbedingungen gegenüber den Reaktionsteilnehmern inerten Lösungs- oder Verteilungsmittels, durch Umsetzung bei Temperaturen zwischen 150°C und dem Siedepunkt des jeweiligen Reaktionsgemisches, vorzugsweise bei Temperaturen zwischen 180 und 220°C, hergestellt werden.

**Revendications pour l'Etat contractant:**

AT

1. Procédé de préparation de composés pyrannohétérocycliques nitrés de formule I:

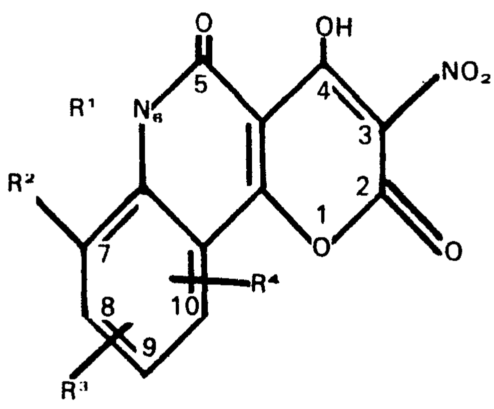

(I)

ainsi que des sels physiologiquement acceptables de ces composés, caractérisé en ce qu'on nitre d'une manière connue en soi des composés de formule:

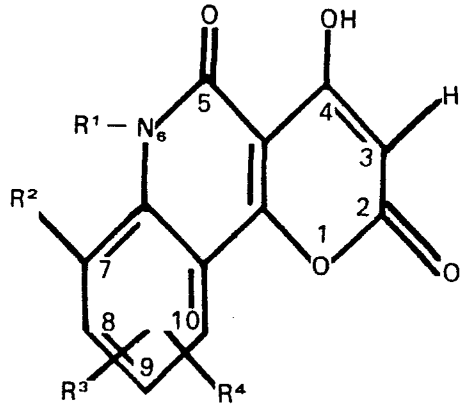

(II)

dans laquelle:

$R^1$ représente l'hydrogène ou un alkyle ayant jusqu'à 4 atomes de carbone,

$R^2$ représente l'hydrogène ou un alkyle, un alcoxy ou un halogénoalkyle ayant chacun jusqu'à 3 atomes de carbone ou un halogène, ou

$R^1$ et $R^2$ forment ensemble un groupe éthylène ou triméthylène, et

$R^3$ et $R^4$ représentent, indépendamment l'un de l'autre, l'hydrogène, un alkyle ou un alcoxy ayant chacun jusqu'à 3 atomes de carbone, qui sont non-substitués, totalement ou partiellement substitués avec des atomes d'halogène identiques ou différents, un halogène ou un alcoxycarbonyle ayant jusqu'à 3 atomes de carbone dans le groupe alkyle, et on transforme éventuellement les composés nitro obtenus avec des bases, en des sels physiologiquement acceptables.

2. Procédé selon la revendication 1, caractérisé en ce qu'on effectue la nitration des composés de formule II dans un mélange d'acide nitrique concentré et d'acide acétique glacial dans un rapport volumique de 1:1 à 1:6 à des températures comprises entre 0 et 110°C.

3. Procédé selon l'une des revendications 1 ou 2, caractérisé en ce qu'on prépare des composés de formule I dans lesquels $R^1$ et $R^2$ forment ensemble un groupe triméthylène, et $R^3$ et $R^4$ sont identiques ou différents et représentent l'hydrogène, un alkyle ou un alcoxy ayant chacun jusqu'à 2 atomes de carbone, un halogène ou le groupe $CF_3$.

4. Procédé selon l'une des revendications 1 ou 2, caractérisé en ce qu'on prépare des composés de formule I dans lesquels $R^1$ représente l'hydrogène ou un alkyle ayant jusqu'à 3 atomes de carbone, $R^2$ représente l'hydrogène, et $R^3$ et $R^4$ sont identiques ou différents et représentent l'hydrogène, un alkyle ou un alcoxy ayant chacun jusqu'à 2 atomes de carbone, un alcoxycarbonyle ayant jusqu'à 3 atomes de carbone dans le groupe alkyle, un halogène ou le groupe $CF_3$.

5. Procédé selon une ou plusieurs des revendications 1 à 4, caractérisé en ce qu'on prépare le sel de sodium du 11-chloro-7,8-dihydro-3-nitro

2,5-dioxo-[2H,5H,9H]-benzo-[ij]-pyranno-[2,3-b]-quinolizine-3-ol.

6. Procédé selon une ou plusieurs des revendications 1 à 5, pour la préparation des sels des composés de formule I, caractérisé en ce qu'on forme les sels alcalins, alcalino-terreux ou d'ammonium physiologiquement compatibles.

7. Procédé selon une ou plusieurs des revendications 1 à 5, caractérisé en ce qu'on prépare la substance de départ de formule II par réaction d'une amine de formule:

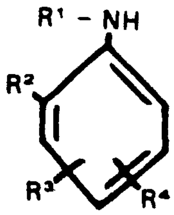

(III)

d'une manière connue en soi, avec au moins une quantité deux fois molaire d'un dérivé de l'acide malonique de formule:

$$R'OOC-CH_2-COOR' \qquad (IV)$$

auquel cas dans les formules II et III, $R^1$, $R^2$, $R^3$ et $R^4$ ont les significations indiquées ci-dessus et R' représente un alkyle ayant jusqu'à 3 atomes de carbone ou un phényle qui peut être substitué jusqu'à 5 fois avec du chlore et/ou du brome.

8. Procédé selon la revendication 7, caractérisé en ce que, pour préparer les composés de départ tétracycliques de formule II où $R^1$ et $R^2$ forment ensemble un pont alkylène, on travaille de préférence sans agent de répartition ou solvant, à des températures comprises entre 150 et 230°C, de préférence entre 190 et 220°C.

9. Procédé selon la revendication 7, caractérisé en ce que, pour préparer les composés de départ tricycliques de formule II, on travaille de préférence en présence d'un solvant ou agent de répartition inerte vis-à-vis des partenaires de réaction dans les conditions de la réaction, à des températures comprises entre 150°C et le point d'ébullition du mélange correspondant, de préférence à des températures comprises entre 180 et 220°C.

**Claims for the contracting State:**
AT

1. Process for the preparation of nitro compounds of pyranoheterocycles of the formula I:

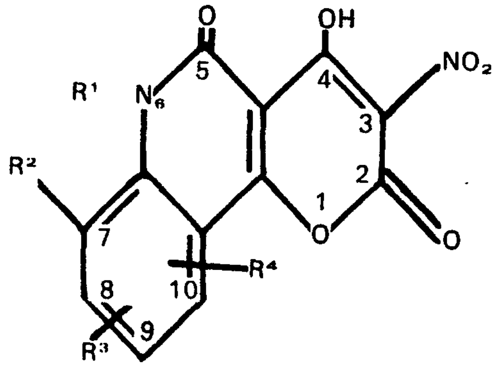

(I)

and their physiologically acceptable salts, characterised in that compounds of formula II:

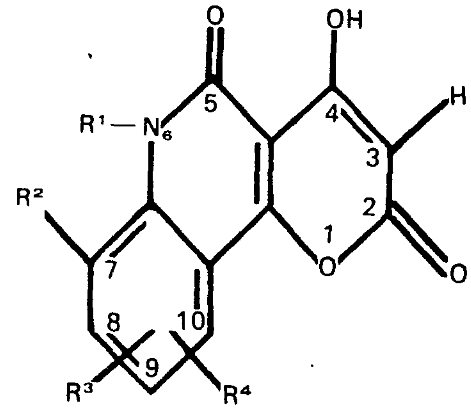

(II)

are nitrated and the obtained nitro compounds are optionally converted with bases into physiologically acceptable salts, wherein, in the above formulae,

$R^1$ is hydrogen or alkyl having 1 to 4 carbon atoms,
$R^2$ is hydrogen, alkyl, alkoxy or haloalkyl each having up to 3 carbon atoms, or halogen, or
$R^1$ and $R^2$ together form an ethylene or trimethylene group, and
$R^3$ and $R^4$, are, independently from each other, hydrogen, alkyl or alkoxy each having up to 3 carbon atoms being unsubstituted, completely or partially substituted, with the same or different halogen atoms; halogen or alkoxycarbonyl having up to 3 carbon atoms in the alkyl group.

2. Process as claimed in claim 1, characterised in that the nitration of the compounds of formula II is performed in a mixture of concentrated nitric acid and glacial acetic acid in a volume ratio of 1:1 to 1:6 at temperatures between 0 and 110°C.

3. Process as claimed in claim 1 or 2, characterised in that such compounds of formula I are prepared, wherein $R^1$ and $R^2$ together form a trimethylene group, and $R^3$ and $R^4$ are identical or different and mean hydrogen, alkyl or alkoxy each with up to 2 carbon atoms, halogen or the $CF_3$-group.

4. Process as claimed in claim 1 or 2, characterised in that such compounds of formula I are prepared, wherein $R^1$ is hydrogen or alkyl with up to 2 carbon atoms, $R^2$ is hydrogen, and $R^3$ and $R^4$ are identical or different and mean hydrogen, alkyl or alkoxy each with up to 2 carbon atoms, alkoxycarbonyl with up to 3 carbon atoms in the alkyl moiety, halogen or the $CF_3$-group.

5. Process as claimed in one or several of claims 1 to 4, characterised in that 11-chloro-7,8-dihydro-3-nitro-2,5-dioxo-[2H,5H,9H]-benzo-[ij]-pyrano-[2,3-b]-quinolizine-4-ol sodium salt is prepared.

6. Process as claimed in one or several claims 1 to 5 for the preparation of salts of the compounds of formula I, characterised in that the physiologically acceptable alkali metal, alkaline earth metal or ammonium salts are formed.

7. Process as claimed in one or several of claims 1 to 5, characterised in that the starting compounds of formula II are prepared by the reaction of an amine of the formula:

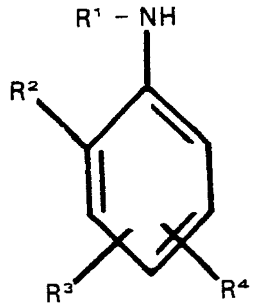

(III)

in a principally known manner, with at least the double molar quantity of a malonic acid derivative of formula:

$$R'OOC-CH_2-COOR' \quad (IV)$$

wherein, in formulae II and III, $R^1$, $R^2$, $R^3$ and $R^4$ have the above-mentioned meanings and R' stands for alkyl with up to 3 carbon atoms or phenyl, which may be substituted up to five times with chlorine and/or cromine.

8. Process as claimed in claim 7, characterised in that the tetracyclic starting compounds of the formula II, in which $R^1$ and $R^2$ together form and alkylene bridge and $R^3$ and $R^4$ have the meanings given in claim 4, are prepared, preferably without dispersing agents or solvents, at temperatures between 150 and 230°C, preferably between 190 and 220°C.

9. Process as claimed in claim 7, characterised in that the tricyclic starting compounds of the formula II are prepared, preferably in the presence of a solvent or dispersing agent which is inert toward the reactants, at temperatures between 150°C and the boiling point of the respective reaction mixture, preferably at temperatures between 180 and 220°C.